# EUROPEAN PATENT SPECIFICATION

(11) **EP 3 841 989 B1**
(45) Date of publication and mention of the grant of the patent: **06.03.2024**
(21) Application number: 20217189.8
(22) Date of filing: 24.12.2020
(51) Int. Cl.: A61B 17/22, A61M 25/00, A61M 25/10

(54) **INTRAVASCULAR CATHETER**
INTRAVASKULÄRER KATHETER
CATHÉTER INTRAVASCULAIRE

(30) Priority: 26.12.2019 US 201916727624
(43) Date of publication of application: 30.06.2021
(73) Proprietor: Neuravi Limited, Galway H91 K5YD (IE)
(72) Inventor: VALE, David, Galway H91 K5YD (IE)
(74) Representative: Carpmaels & Ransford LLP

(56) References cited:
- CN-Y- 200 998 342
- JP-A- H09 103 491
- US-A- 5 843 022
- US-A1- 2010 222 736
- US-A1- 2012 253 284
- US-A1- 2013 165 944
- US-A1- 2018 235 743
- US-B1- 6 544 223

## Description

### BACKGROUND OF THE INVENTION

### Field of the Invention

The present invention relates to an intravascular catheter and, in particular, to an intravascular catheter for capture and retrieval of a thrombus or clot or other material occluding flow through a blood vessel.

### Description of Related Art

Acute ischemic stroke is primarily caused by a thrombotic or embolic occlusion (e.g., blockage) in an artery of the brain. The occlusion is typically caused by a blood clot liberated from another part of the body which travels in an antegrade direction (in the direction of normal blood flow) through the vessel and eventually becomes lodged in a neurovascular artery, where it obstructs blood flow to a region of the brain.

A procedure known as a thrombectomy may be used to remove the thrombus, occlusion, blockage or clot lodged in the vessel using a mechanical retrieval device. During the thrombectomy procedure or treatment a physician or interventionalist endovascularly introduces a guidewire and microcatheter together through the vasculature, typically in an artery located in the groin or the arm or by direct access through the carotid artery. Together the guidewire and microcatheter are advanced to a location facing a proximal side of the targeted clot, blockage or occlusion. Then the guidewire is advanced across the clot, followed by the microcatheter. While in a compressed state, a mechanical thrombectomy device may be guided through the lumen of the microcatheter to the target site. Upon emerging from the microcatheter the mechanical thrombectomy device typically automatically expands to its original enlarged state. Mechanical thrombectomy devices are typically made of a selfexpanding biocompatible material such as nickel-titanium. Aspiration through the catheter may accompany or be used in place of the mechanical retrieval device to remove the clot.

During retrieval of the blockage it is preferable for the distal end or tip of the catheter to be brought as close as possible to the outer face or surface of the thrombus, occlusion, blockage or clot. To enhance maneuverability of the catheter through tortuous vasculature pathways while reducing trauma to the tissue of the inner wall of the vessel it is desirable to minimize the friction (i.e., low friction) therebetween. Typically, friction is reduced by applying a hydrophilic coating or other lubricious coating such as silicone, Polytetrafluoroethylene (PTFE), polyvinylpyrrolidone (PVP) to the exterior surface of the catheter. However, the use of such low friction coatings may have one or more associated drawbacks, for example, readily spreading to undesirable areas and/or degrading/breaking-off of unwanted downstream particulate. A low friction surface on an interior surface of the distal portion of the catheter would also be desirable to minimize the shear forces on the clot as it is ingested by the catheter. Furthermore, it may be desirable to enlarge/expand in diameter the distal tip or end of the clot retrieval catheter to optimize full clot ingestion resulting in higher first-pass successful recanalization. Simply enlarging or expanding the distal tip may increase the rigidity of the catheter thus undesirably hampering the flexibility beneficial during navigation through the vessel to the target site. It is therefore desirable to develop an improved intravascular catheter that overcomes these aforementioned shortcomings.

US 2010/222736 A1 relates to a catheter system comprising a guidewire, an endovascular catheter, and an aspiration catheter. The guidewire has an expandable occluder mounted on a distal end. The guidewire and the endovascular catheter are insertable into a lumen of the aspiration catheter.

US 5843022 A1 relates to an intravascular device and associated system which utilizes pressurized fluid to extract occlusive material. The device includes independently movable fluid input and fluid output tubes and may be advanced over a guide wire. The fluid port holes are located immediately adjacent the distal end of the fluid output tube so as to engage the occlusive material without the need to first traverse the occlusive material with the device.

US 2012/253284 A1 relates to devices that include a conduit and a distal head region located at the distal end of said conduit. A plurality of apertures on the surface of said head region and conduit may be used to direct a liquid spray outwards from said device.

US 2013/165944 A1 relates to systems and methods for removing an obstruction from a ureter using a catheter including a distal end sized for introduction into a ureter, an infusion lumen, and an aspiration lumen. An infusion tip is provided on the distal end that includes infusion ports communicating with the infusion lumen, and one or more aspiration ports communicating with the aspiration lumen.

US 6544223 B 1 relates to a drug delivery device for delivering therapeutic agents and method of making such a device. The device including an inflatable balloon having a plurality of holes formed in the wall of the balloon. A microporous coating covers a portion of the outer surface of the wall of the balloon.

### Summary of the Invention

The invention is defined in claim 1. Embodiments of the invention are defined in the dependent claims. Methods as such do not form part of the claimed subject matter.

Still another possible aspect of the present invention relates to an intravascular catheter wherein at least a portion of a distal section of the catheter is enlargeable/expandable to increase in diameter so as to physically contact and form a seal with an inner wall of the vessel thereby arresting blood flow therethrough.

Still another aspect of the present invention relates to assisting advancement of the catheter through the vasculature by directing jets of fluid through orifices in the distal porous cuff section/tip of the catheter in a retrograde direction.

Still another aspect of the present invention relates to lowering the coefficient of friction of the inner surface of a distal porous cuff section/tip of the catheter.

The present invention, as defined by claim 1, is directed to an intravascular catheter including a distal porous cuff section having a plurality of unobstructed openings defined radially outward from an annular inflation chamber, wherein the distal porous cuff section is non-rotatable. The catheter also including a tubular body disposed proximally of the distal porous cuff section, the tubular body having a sidewall extending from a proximal end to the distal porous cuff section with an inner lumen defined axially therethrough. The sidewall of the tubular body having an inflation supply channel extending in a direction parallel to an axial direction of the intravascular catheter.

In addition, the present disclosure also describes a non claimed method for using an intravascular catheter having a proximal end and an opposite distal end. The intravascular catheter includes a distal porous cuff section having a plurality of unobstructed openings defined radially outward from an annular inflation chamber, wherein the distal porous cuff section is non-rotatable. The intravascular catheter also having a tubular body disposed proximally of the distal porous cuff section, the tubular body having a sidewall extending from a proximal end to the distal porous cuff section with an inner lumen defined axially therethrough. The sidewall of the tubular body having an inflation supply channel extending in a direction parallel to an axial direction of the intravascular catheter. The present inventive method including the step of while navigating the intravascular catheter so that the distal end is proximate a clot in a vessel, minimizing friction between an outer surface of the intravascular catheter and an inner wall of the vessel by injecting pressurized fluid into the inflation supply channel generating a cuff of fluid jets discharged from the plural openings defined in the distal porous cuff section of the tubular body and into the vessel.

### Brief Description of the Drawing

The foregoing and other features of the present invention will be more readily apparent from the following detailed description and drawings illustrative of the invention wherein like reference numbers refer to similar elements throughout the several views and in which:
Figure 1 is a cross-sectional view of a portion of a vessel showing a distal porous cuff section/tip of the present inventive intravascular catheter disposed in close proximity to a target clot;
Figure 2 is a side view of a first embodiment of the present inventive intravascular catheter of Figure 1, wherein the tubular body of the catheter proximal of the distal porous cuff section/tip is configured as a single wall with a single axial inflation supply channel defined therein;
Figure 2A is a cross-sectional view of the intravascular catheter of Figure 2 along the lines II(A) -II(A) through the distal porous cuff section/tip;
Figure 2B is a cross-sectional view of the intravascular catheter of Figure 2 along the lines II(B) -II(B) through the tubular body proximal of the distal porous cuff section/tip;
Figure 2C is a cross-sectional view through the intravascular catheter of Figure 2 along lines II(C) - II(C) including the distal porous cuff section/tip;
Figure 2D is a radial cross-sectional view proximal to the distal porous cuff section/tip depicting an alternative configuration of the tubular body of the present inventive intravascular catheter having a single wall sidewall, similar to that of Figure 2B, but with multiple axial inflation supply channels defined therein;
Figure 3A is a longitudinal cross-sectional view of the distal porous cuff section/tip of the present inventive intravascular catheter depicting the fluid discharged radially outward through openings in fluid communication with the annular inflation chamber, wherein those openings are arranged non-perpendicularly (i.e., at an inclined angle facing a proximal end of the catheter relative to that of the axial direction of the catheter);
Figure 3B is a longitudinal cross-sectional view of the distal porous cuff section/tip of the present inventive intravascular catheter depicting the fluid discharged radially outward through openings in fluid communication with the annular inflation chamber, wherein those openings are arranged non-perpendicularly (i.e., at an inclined angle facing a distal end of the catheter relative to that of the axial direction of the catheter);
Figure 4 is a longitudinal cross-sectional view of the distal porous cuff section/tip of the present inventive intravascular catheter depicting the fluid discharged both radially outward and radially inward through openings in fluid communication with the annular inflation chamber;
Figure 5A depicts one configuration of the distal porous cuff section/tip of the present inventive intravascular catheter in an enlarged/expanded bulging state;
Figure 5B depicts the distal porous cuff section of the present inventive intravascular catheter of Figure 5A in a non-enlarged/non-expanded state;
Figure 5C is a cross-sectional view of the tubular body proximal to the distal porous cuff section/tip along lines V(C) -V(C) of Figure 5B depicting the single radial section inflation supply channel defined in the single wall of the tubular body of the intravascular catheter;
Figure 6A depicts a longitudinal cross-sectional view of the distal porous cuff section/tip of another configuration of the present disclosure depicting an intravascular catheter in a flared state forming a conical funnel illustrating the internal connecting ribs disposed in the annular inflation chamber;
Figure 6B is a side view of the distal porous cuff section/tip of the intravascular catheter of Figure 6A in a non-flared state;
Figure 7 is a side view of still another configuration of the present inventive intravascular catheter, wherein the tubular body proximal of the distal porous cuff section/tip is configured as two concentric walls separated radially a predetermined distance from one another to define an annular inflation supply channel therebetween;
Figure 7A is a cross-sectional view of the intravascular catheter of Figure 7 along the lines VII(A) -VII(A) through the distal porous cuff section/tip;
Figure 7B is a cross-sectional view of the intravascular catheter of Figure 7 along the lines VII(B) -VII(B) through the tubular body proximal of the distal porous cuff section/tip; and
Figure 7C is a cross-sectional view of the intravascular catheter of Figure 7 along lines VII(C) - VII(C) including the distal porous cuff section/tip of the intravascular catheter.

### Detailed Description of the Invention

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist. The terms "occlusion", "clot" or "blockage" are used interchangeably.

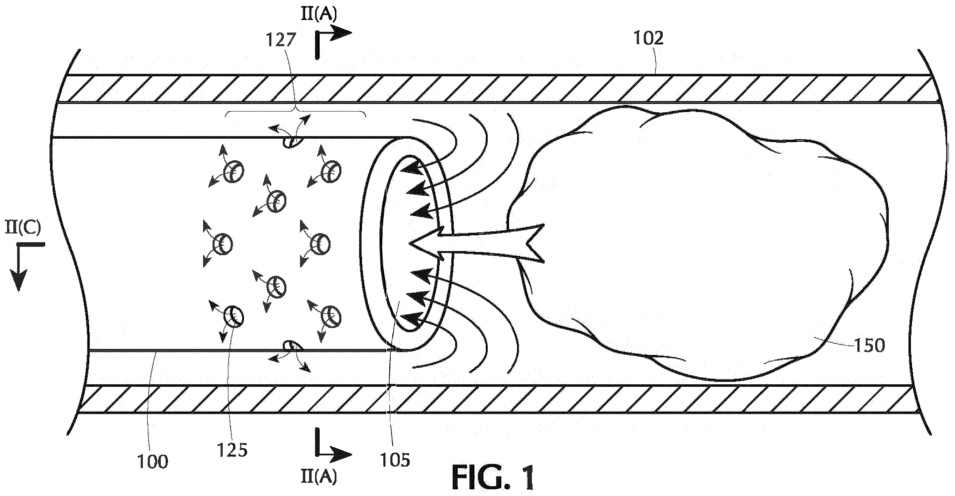

Figure 1 depicts a partial axial cross-sectional view through a vessel 102 having a clot, blockage, thrombus, or occlusion 150 lodged therein. Advanced close up to the clot 150 is the present inventive intravascular catheter 100 having an inner central lumen 105 defined axially therethrough from its proximal end to an opposite distal end. The distal and proximal ends of the inner central lumen 105 being open and sized to receive therethrough the captured clot during removal. An auxiliary device, for example, guide wire, delivery wire, mechanical thrombectomy device, receiving catheter, etc. may be advanced in a distal direction through the inner central lumen 105. A biocompatible fluid, preferably saline, is injectable under pressure exclusively via one or more inflation supply channels defined in the tubular body of the catheter and arranged radially outward of the inner central lumen 105.

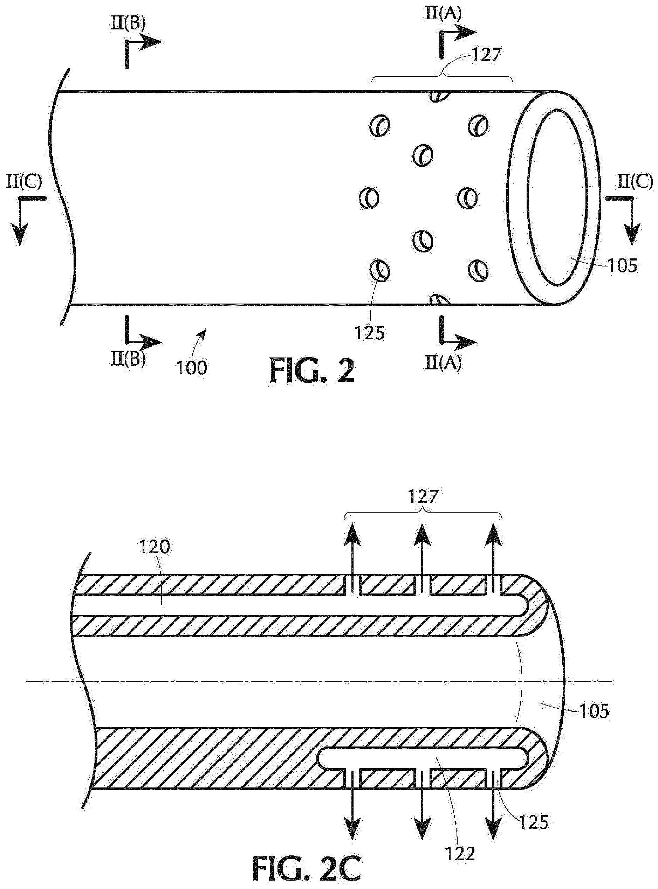

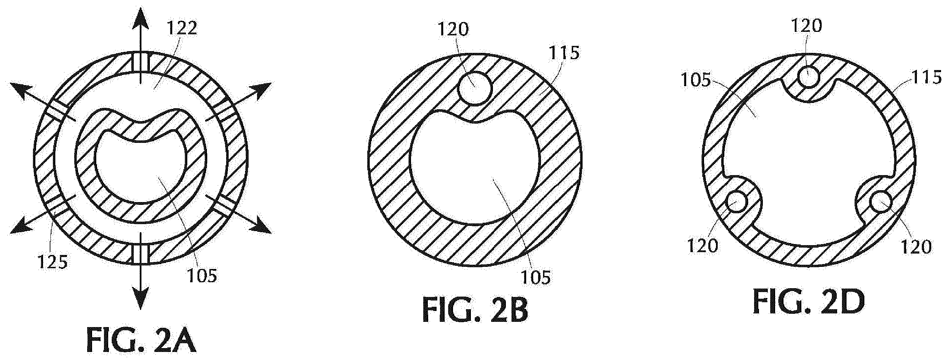

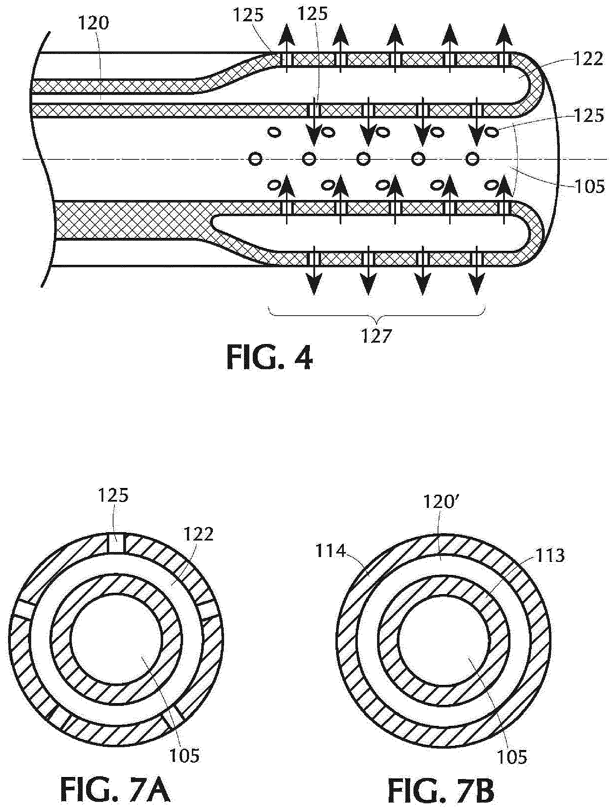

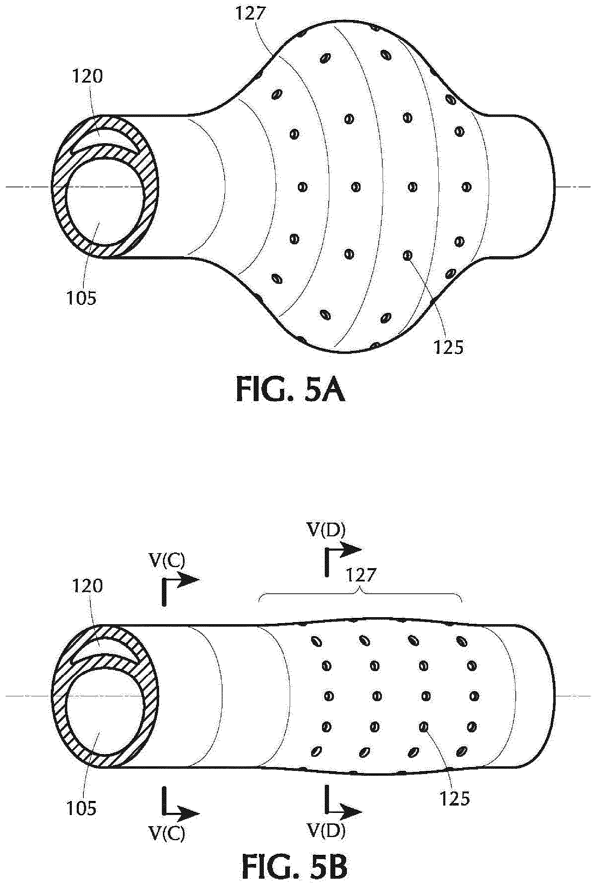

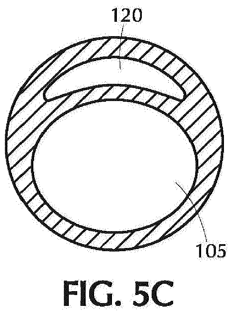

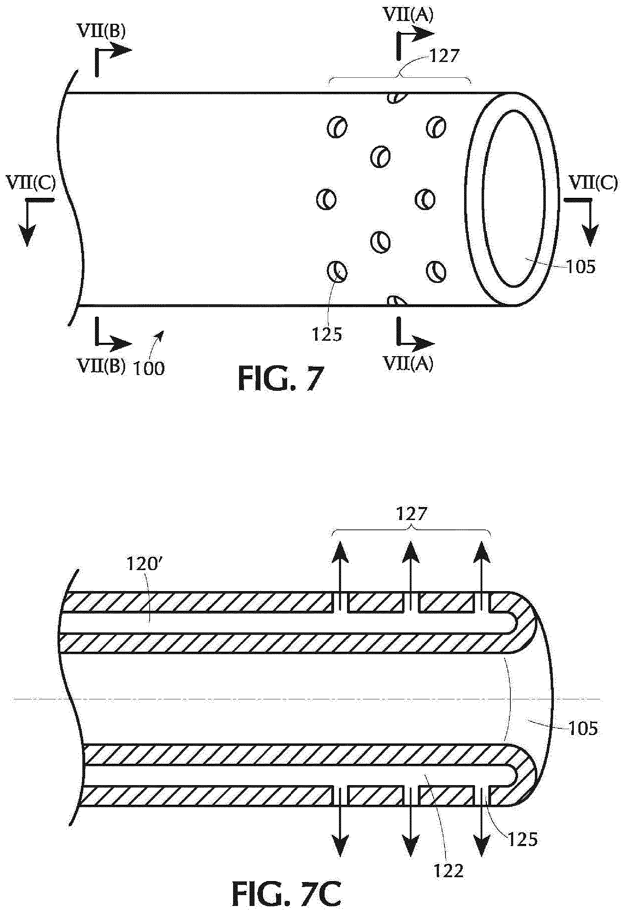

Figure 2 shows a side view of a distal portion of a first embodiment of the present inventive intravascular catheter including a distal porous cuff section/tip 127 and a tubular body (i.e., the remaining portion of the intravascular catheter disposed proximally of the distal porous cuff section/tip). In this first embodiment, a sidewall 115 of the tubular body is configured as a single wall comprising either a single layer or a laminate/composite of multiple layers (e.g., liner, braid, polymer jacket and/or coating). A single inflation supply channel 120 extending parallel to an axial direction of the catheter if formed or defined (e.g., molded) in the single wall sidewall 115. Figure 2B shows a cross-sectional view along lines II(B) - II(B) in Figure 2 through the tubular body proximal of the distal porous cuff section/tip 127 of the catheter. Only a single axial inflation channel 120 is shown in Figure 2B in a single wall sidewall, whereas Figure 2D depicts an alternative design of the single wall sidewall 115 of the tubular body of the catheter having three axial inflation supply channels 120 defined therein, each axial inflation channel being separated radially equidistantly from one another. Any number of one or more axial inflation supply channels 120 as well as the diameter and the arrangement of such axial inflation supply channels in the single wall sidewall of the tubular body of the catheter may be modified, as desired. It is further contemplated that a radial section inflation supply channel may be formed in the single wall of the tubular body of the present inventive intravascular catheter, as seen in Figure 5C. A two-walled tubular body construction is also possible wherein the inflation supply channel is defined by the space between the two walls of a two-wall catheter, as shown in Figures 7-7D, and described in greater detail below.

A distal section of the intravascular catheter 100 serves as a fixed (non-rotating relative to the remaining tubular body proximal thereto) distal porous cuff section/tip 127 having an annular inflation chamber 122 in fluid communication with the one or more inflation supply channel(s) (axial or radial) 120. As is clearly illustrated in Figure 2A, a plurality of opening 125 are defined in the distal porous cuff section/tip 127 arranged radially outward from the annular inflation chamber 122. Preferably, openings 125 are arranged 360 degrees in the distal porous cuff section/tip 127. The exemplary axial cross-sectional view in Figure 2C shows the annular inflation chamber 122 of the distal porous cuff section/tip 127 in fluid communication with the single axial inflation supply channel 120 of the tubular body, as well as the plural openings 125 defined radially outward and in fluid communication with the annular inflation chamber 122. Dispensed under pressure via the one or more axial inflation supply channel(s) 120, a biocompatible fluid (e.g., saline) emerging from openings 125 defined in the distal porous cuff section/tip 127 produces a "cuff", "cloud", "pillow", "pool" of fluid jets about a distal section of the outer perimeter of the catheter that minimizes friction with the vessel wall, minimizes damage to the surrounding tissue, and aids in navigation allowing the distal end or tip of the catheter to be advanced close up to the clot face. The "cuff' of fluid jets produced from the inflation fluid being injected at a pressure sufficient to produce a fluid boundary between the outer surface of the catheter and the inner wall of the vessel to minimize friction therebetween, without degrading or breaking-up the clot. The arrangement of the openings 125 in the distal porous cuff section/tip 127 of the catheter ensures that the clot is not degraded or broken-up by the fluid jets. During discharge of the fluid from the openings 125, the distal porous cuff section/tip 127 remains stationary, fixed or non-rotating relative to the tubular body of the catheter proximal thereto. Radial openings 125 in the distal porous cuff section/tip 127 of the catheter serve exclusively as discharge ports for the passage of fluid from the catheter. At no time is fluid and/or material externally of the catheter introduced internally into the catheter via the radial openings 125. In other words, the fluid once discharged through the radial openings 125 is not thereafter circulated back through the radial openings (either the same radial opening or any other radial opening along the sides of the catheter). Moreover, all of the radial openings along the sides of the catheter remain unobstructed and exteriorly free of any covering extending over the openings.

In an alternative configuration shown in Figure 7, the tubular body proximal of the distal porous cuff section/tip of the present inventive catheter may have a two-wall sidewall construction, rather than the single wall construction depicted in Figure 2. Figures 7A & 7B show the two-wall catheter construction of Figure 7 along lines VII(A)-VII(A) (through the distal porous cuff section/tip) and VII(B)-VII(B) (through the tubular body proximal to the distal porous cuff section/tip), respectively. The two-wall sidewall construction of the tubular body proximal of the distal porous cuff section/tip 127 is shown in Figure 7B including an inner wall 113 disposed radially inward of an outer wall 114 separated a predetermined distance from one another to form an annular, radial, circular, ring-shaped or circumferential inflation supply channel 120' therebetween. Inner and outer walls 113, 114, respectively, are preferably concentric, as shown in Figure 7B, but may otherwise be eccentrically arranged. Once again, a plurality of openings 125 are defined radially outward from the annular inflation chamber 122 of the distal porous cuff section/tip 127 (Figure 7A).

In the case of the two-wall tubular body construction of the present inventive intravascular catheter (as shown in Figure 7B) forming an annular inflation supply channel 120' between the walls, the fluid introduced is dispensed substantially equally from the openings 125 of the distal porous cuff section/tip as fluid jets. Whereas in the case of one or more axial inflation channel(s) 120 (Figures 2B or 2D) formed in a single wall of the tubular body of the catheter, the fluid introduced is dispensed from the openings 125 of the distal porous cuff section/tip unequally. That is the fluid may be dispensed at a faster rate from those openings 125 closest to the proximal end of the catheter relative to those openings 125 closest to the opposite distal end of the catheter. The diameter of the openings 125 may be varied to substantially equalize the dispensing of fluid from those openings 125.

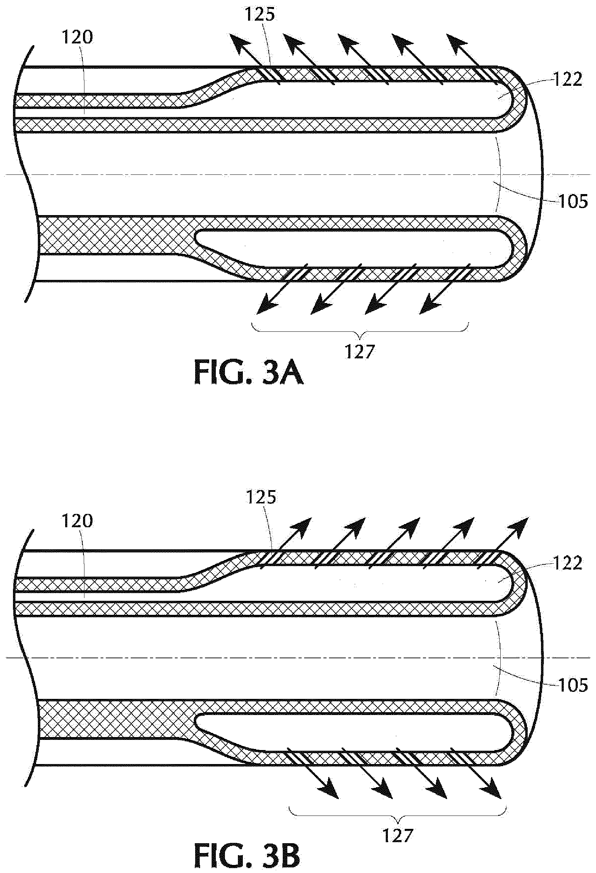

Referring to Figure 2C, the openings 125 defined radially outward in the distal porous cuff section/tip 127 may be arranged perpendicular to the axial direction of the catheter. These openings 125 in the distal porous cuff section/tip may alternatively be arranged at a non-perpendicular angle (i.e., inclined angle). In an exemplary axial cross-sectional view through the distal porous cuff section/tip shown in Figure 3A, the openings 125 in the distal porous cuff section/tip are defined radially outward at a non-perpendicular (i.e., an inclined angle facing proximal towards the proximal end of the catheter) so that the fluid jets produced push/advance the catheter forward in a distal direction to assist maneuvering of the catheter through the vessel to the desired location. Still another non-perpendicular (i.e., inclined angle facing distal towards the distal end of the catheter) arrangement of the openings 125 defined radially outward in the distal porous cuff section/tip is shown in Figure 3B. Arranging the openings to face distal (Figure 3B) supplies additional fluid to the space between catheter and clot, which creates a "pool" or "cloud" of fluid to surround the clot and enter the catheter tip under aspiration. This fluid can also serve to reduce the friction between clot and catheter. Still further, the openings need not all conform, in that, some may be arranged perpendicularly whereas others may be arranged non-perpendicularly (i.e., inclined angle facing proximal and/or distal) relative to the axial direction of the catheter.

In Figure 2C, openings 125 are defined only radially outward from the annular inflation chamber 122 in the distal porous cuff section/tip 127 to assist in advancing the catheter through the vessel to the target site. It is further contemplated that the openings 125 may also be defined radially inward from the annular inflation chamber 122 in the distal porous cuff section/tip 127 to aid drawing or ingesting the captured clot into the catheter during aspiration. Figure 4 depicts the distal porous cuff section/tip wherein perpendicularly arranged openings 125 are defined both radially inward and radially outward from the annular inflation chamber 122. Accordingly, openings 125 may be defined radially inward from and/or radially outward from the annular inflation chamber 122 in the distal porous cuff section/tip 127 and at any desired arrangement (e.g., perpendicular, non-perpendicular/inclined angle) of such openings. The angle of arrangement need not be same among the openings.

As an alternative to use of a separate conventional inflatable balloon component joined, connected, attached or welded to the outer surface of a catheter to occlude blood flow, the annular inflation chamber 122 of the distal porous cuff section/tip 127 of the present inventive intravascular catheter may achieve this goal without obstructing, isolating or covering any of the radial openings 125. Specifically, if the pressure and/or volume of the fluid being introduced into the annular inflation chamber 122 exceeds the rate at which it can be dispensed through the radial openings 125 then the pressure and volume of fluid introduced into the annular inflation chamber 122 increases/builds altering the outer contour of the distal porous cuff section/tip 127 (i.e., hyper inflated state). In one configuration shown in Figures 5A-5D, an outer wall of distal porous cuff section/tip 127 is made of a compliant material. Aside from at the distal end of the annular inflation chamber 122, internally within the annular inflation chamber 122 the inner and outer radial walls separated from one another by a predetermined distance are not secured, joined or tethered to one another in any way (i.e., the annular inflation chamber is devoid of any internal connecting ribs). Thus, as the pressure and volume of fluid injected/introduced into the annular inflation chamber 122 increases/builds, the compliant outer wall bulges radially outward. If sufficient pressure increases/builds in the annular inflation chamber 122, the expanded or enlarged diameter of the outer wall of the distal porous cuff section will come into physical contact with and seal against an inner wall of the vessel arresting blood flow therethrough. Because the annular inflation chamber 122 is devoid of any internal connecting ribs inflation with the highly pressurized fluid produces a radially outward bulging contour of the outer wall of the distal porous cuff section/tip 127 (similar to an inflated balloon) relative to that of the inner wall.

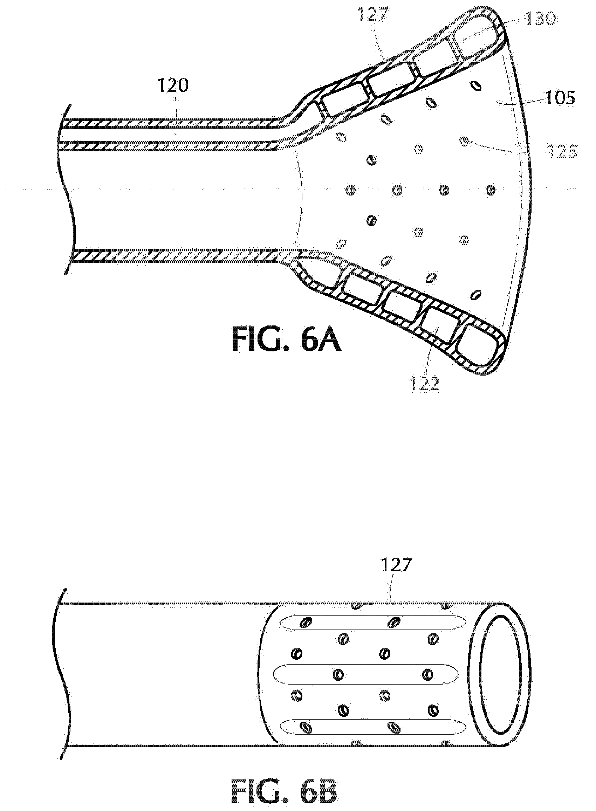

Rather than a bulging outer contour, the distal porous cuff section/tip 127 may alternatively be configured so that when subject to injection of the fluid into the annular inflation chamber 122 at a pressure and volume greater than what can be dispensed through the openings 125 the distal porous cuff section/tip flares outward to form a conical funnel shape. An axial cross-sectional view of the distal cuff section/tip of the catheter in an enlarged/expanded state having a flared conical funnel shape, not in accordance with the present claimed invention, is shown in Figure 6A, while its non-enlarged/non-expanded state is shown in Figure 6B. The largest diameter of the flared conical funnel shaped distal porous cuff section/tip presiding at the distal most end with the opposite proximal end having a diameter substantially equal to that of the remaining tubular body of the catheter proximal to the distal porous cuff section/tip. In a non-flared state, the diameter (both inner and outer diameters) of the distal porous cuff section/tip is substantially equal to that of the diameter (both inner and outer diameters) of the remaining tubular body section of the catheter proximal to the distal porous cuff section/tip. Flaring of the distal porous cuff section/tip into a conical funnel shape is achieved by having a plurality of connecting ribs 130 disposed internally within the annular inflation chamber 122 itself (similar to that of an air bed). As a result of the internal connecting ribs 130, as the volume and pressure of the fluid in the annular inflation chamber 122 increases (rather than the outer wall bulging like a balloon) the inner and outer walls remain substantially equidistant to one another while the distal porous cuff section/tip 127 flares into a conical funnel shape. Advantageously, this alternative conical funnel shape configuration serves a dual purpose of both occluding blood flow and the enlarged diameter of the distal end optimizes complete ingestion of the clot into the catheter.

Instead of requiring application of suction or negative pressure to deflate as is required with a conventional balloon sealed to the outer surface of the catheter, the bulge or flared conical funnel produced by the introduction of the highly pressurized fluid into the annular inflation chamber of the distal porous cuff section/tip of the present inventive intravascular catheter advantageously deflates itself automatically. Hence the need for the application of suction or negative pressure to deflate the outer contour of the distal porous cuff section/tip has been eliminated. That is, cessation or decrease in pressure of the fluid injected into the annular inflation chamber 122 results in a natural automatic self-deflation of the pressurized fluid therein via the openings 125 and contraction/reduction of the diameter of the outer contour of the distal porous cuff section/tip until equilibrium pressure is realized outside the catheter and within the annular inflation chamber 122.

In operation, to aid maneuverability while the intravascular catheter navigates through the vessel to a site proximate a target clot, a biocompatible fluid, preferably saline, is injected under positive pressure through the one or more inflation channels 120, 120' defined in the sidewall of the tubular body of the present inventive intravascular catheter. The pressurized fluid enters the annular inflation chamber 122 and is discharged (as fluid jets) through the plural openings 125 defined radially outward and/or radially inward from the annular inflation chamber 122 of the distal porous cuff section/tip 127. The "cuff'," cloud" or "pillow" of fluid jets produced out from the openings of the distal porous cuff section/tip of the present inventive intravascular catheter minimizes friction and reduces the risk of irritation or trauma to the surrounding tissue. Due to the low friction "cuff", "cloud" or "pillow" created by the fluid jets the distal end of the intravascular catheter is able to be maneuvered up close to the face or surface of the clot. If openings are defined radially inward from the annular inflation chamber 122 in the distal porous cuff section/tip, then the fluid jets produced therefrom in the inner central lumen 105 assist complete ingestion of the clot into the catheter.

Once the distal end of the catheter is positioned proximal to the clot, a vacuum (e.g., negative pressure) may be applied to the inner central lumen 105 to retrieve the clot into the catheter via aspiration. To optimize complete and full ingestion of the clot, the diameter of the distal end of the catheter is preferably larger than the diameter of the clot. However, an expanded or enlarged diameter is undesirable during navigation of the tortuous pathway. Accordingly, the present inventive intravascular catheter has a normal (non-enlarged/non-expanded) outer diameter size distal porous cuff section/tip when advanced to the target site within the vessel proximate the proximal side of the clot. Once properly positioned, the same inflation channel used for introduction of the biocompatible fluid under pressure may also be used to transition the outer contour of the distal porous cuff section of the catheter from a first state having a non-enlarged/non-expanded diameter, to a second state having an enlarged/expanded diameter to more easily accommodate the clot therein thereby optimizing complete ingestion of the clot. If while in an enlarged/expanded state, a portion of outer surface of the distal porous cuff section physically contacts with an inner wall of the vessel forming a seal therebetween an additional benefit may be realized of occluding blood flow in the vessel.

The "cuff', "cloud" or "pillow" of fluid jets produced about the distal porous cuff section/tip of the present inventive intravascular catheter reduces surface friction with that of the inner wall of the vessel during deliverability of the catheter to a target site. Accordingly, the present inventive intravascular catheter, in some applications, may eliminate altogether the need for a low friction lubricious coating to be applied to the outer surface of the catheter. However, it is contemplated and within the present scope of the invention for the present inventive catheter to also employ a low friction coating.

Thus, while there have been shown, described, and pointed out fundamental novel features of the invention as applied to a preferred embodiment thereof, it will be understood that various omissions, substitutions, and changes in the form and details of the systems/devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. An intravascular catheter (100) having a proximal end and an opposite distal end, the intravascular catheter comprising:
a distal porous cuff section (127) having a plurality of unobstructed openings (125) defined radially outward from an annular inflation chamber; the distal porous cuff section being non-rotatable; and
a tubular body disposed proximally of the distal porous cuff section, the tubular body having a sidewall extending from a proximal end to the distal porous cuff section with an inner lumen (105) defined axially therethrough; the sidewall of the tubular body having an inflation supply channel (120) extending in a direction parallel to an axial direction of the intravascular catheter; and
wherein the annular inflation chamber has no internal connecting ribs; and in a hyper inflated state at least a portion of an exterior surface of the distal porous cuff section is bulgeable radially outward; and
wherein the distal porous cuff section and the tubular body are integrally formed.

2. The intravascular catheter according to claim 1, wherein the sidewall of the tubular body is a single wall comprising a single layer or a laminate of multiple layers; and the inflation supply channel is molded in the single wall.

3. The intravascular catheter according to claim 1, wherein the sidewall comprises two walls including an inner wall and an outer wall; and the inflation supply channel is an annular inflation supply channel defined between the two walls separated radially a predetermined distance from one another.

4. The intravascular catheter according to claim 1, wherein at least some of the plural openings in the distal porous cuff section are arranged perpendicular to an axial direction of the intravascular catheter.

5. The intravascular catheter according to claim 1, wherein at least some of the plural openings in the distal porous cuff section are arranged at a non-perpendicular angle to an axial direction of the intravascular catheter; wherein the non-perpendicular angle is an inclined angled towards the proximal end of the intravascular catheter or an inclined angle towards the distal end of the intravascular catheter.

6. The intravascular catheter according to claim 1, wherein the distal porous cuff section has a plurality of openings defined radially inward in fluid communication between the annular inflation chamber and the inner lumen.

7. The intravascular catheter according to claim 1, wherein the plural openings remain uncovered exteriorly.

## Patentansprüche

1. Intravaskulärer Katheter (100) mit einem proximalen Ende und einem gegenüberliegenden distalen Ende, wobei der intravaskuläre Katheter umfasst:
einen distalen porösen Manschettenabschnitt (127) mit einer Vielzahl von freien Öffnungen (125), die von einer ringförmigen Aufblaskammer radial nach außen definiert sind; wobei der distale poröse Manschettenabschnitt nicht drehbar ist; und
einen röhrenförmigen Körper, der proximal zu dem distalen porösen Manschettenabschnitt angeordnet ist, wobei der röhrenförmige Körper eine Seitenwand hat, die sich von einem proximalen Ende zu dem distalen porösen Manschettenabschnitt erstreckt, durch ein Innenlumen (105), das axial dort hindurch definiert ist; wobei die Seitenwand des röhrenförmigen Körpers einen Aufblaskanal (120) hat, der sich in eine Richtung parallel zu einer axialen Richtung des intravaskulären Katheters erstreckt; und
wobei die ringförmige Aufblaskammer keine inneren Verbindungsrippen hat und in einem stark aufgeblasenen Zustand mindestens ein Abschnitt einer Außenseite des distalen porösen Manschettenabschnitts radial nach außen wölbbar ist; und
wobei der distale poröse Manschettenabschnitt und der röhrenförmige Körper einstückig gebildet sind.

2. Intravaskulärer Katheter nach Anspruch 1, wobei die Seitenwand des röhrenförmigen Körpers eine Einzelwand ist, die eine Einzelschicht oder ein Verbund aus mehreren Schichten ist; und der Aufblaskanal in der Einzelwand eingeformt ist.

3. Intravaskulärer Katheter nach Anspruch 1, wobei die Seitenwand zwei Wände umfasst, die eine Innenwand und eine Außenwand aufweisen, und der Aufblaskanal ein ringförmiger Aufblaskanal ist, der zwischen den zwei Wänden definiert ist, die einen vorbestimmten Abstand radial voneinander getrennt sind.

4. Intravaskulärer Katheter nach Anspruch 1, wobei mindestens einige der mehreren Öffnungen in dem distalen porösen Manschettenabschnitt senkrecht zu einer axialen Richtung des intravaskulären Katheters angeordnet sind.

5. Intravaskulärer Katheter nach Anspruch 1, wobei mindestens einige der mehreren Öffnungen in dem distalen porösen Manschettenabschnitt in einem nicht rechten Winkel zu einer axialen Richtung des intravaskulären Katheters angeordnet sind, wobei der nicht rechte Winkel ein in Richtung des proximalen Endes des intravaskulären Katheters geneigter Winkel oder ein in Richtung des distalen Endes des intravaskulären Katheters geneigter Winkel ist.

6. Intravaskulärer Katheter nach Anspruch 1, wobei der distale poröse Manschettenabschnitt eine Vielzahl von Öffnungen hat, die radial nach innen in Fluidverbindung zwischen der ringförmigen Aufblaskammer und dem Innenlumen definiert sind.

7. Intravaskulärer Katheter nach Anspruch 1, wobei die mehreren Öffnungen außen frei bleiben.

## Revendications

1. Cathéter intravasculaire (100) ayant une extrémité proximale et une extrémité distale opposée, le cathéter intravasculaire comprenant :
une section de manchon poreuse distale (127) présentant une pluralité d'ouvertures non obstruées (125) définies radialement vers l'extérieur à partir d'une chambre de gonflage annulaire ; la section de manchon poreuse distale n'étant pas rotative ; et
un corps tubulaire disposé à proximité de la section de manchon poreuse distale, le corps tubulaire ayant une paroi latérale s'étendant d'une extrémité proximale à la section de manchon poreuse distale avec une lumière interne (105) définie axialement à travers elle ; la paroi latérale du corps tubulaire ayant un canal d'alimentation de gonflage (120) s'étendant dans une direction parallèle à une direction axiale du cathéter intravasculaire ; et
la chambre de gonflage annulaire n'ayant pas de nervures de connexion internes ; et dans un état hyper gonflé, au moins une partie d'une surface extérieure de la section de manchon poreuse distale étant bombée radialement vers l'extérieur ; et
la section de manchon poreuse distale et le corps tubulaire étant formés d'un seul tenant.

2. Cathéter intravasculaire selon la revendication 1, la paroi latérale du corps tubulaire étant une paroi unique comprenant une seule couche ou un stratifié de couches multiples ; et le canal d'alimentation en air étant moulé dans la paroi unique.

3. Cathéter intravasculaire selon la revendication 1, la paroi latérale comprenant deux parois, dont une paroi interne et une paroi externe ; et le canal d'alimentation en gaz étant un canal d'alimentation en gaz annulaire défini entre les deux parois séparées radialement d'une distance prédéterminée l'une de l'autre.

4. Cathéter intravasculaire selon la revendication 1, au moins certaines de la pluralité d'ouvertures dans la section de manchon poreuse distale étant disposées perpendiculairement à une direction axiale du cathéter intravasculaire.

5. Cathéter intravasculaire selon la revendication 1, au moins certaines de la pluralité d'ouvertures dans la section de manchon poreuse distale étant disposées à un angle non perpendiculaire à une direction axiale du cathéter intravasculaire ; l'angle non perpendiculaire étant un angle incliné vers l'extrémité proximale du cathéter intravasculaire ou un angle incliné vers l'extrémité distale du cathéter intravasculaire.

6. Cathéter intravasculaire selon la revendication 1, la section de manchon poreuse distale ayant une pluralité d'ouvertures définies radialement vers l'intérieur en communication fluidique entre la chambre de gonflage annulaire et la lumière interne.

7. Cathéter intravasculaire selon la revendication 1, la pluralité d'ouvertures restant découvertes à l'extérieur.
